# EUROPÄISCHE PATENTSCHRIFT

(11) **EP 1 959 013 B1**
(45) Veröffentlichungstag und Bekanntmachung des Hinweises auf die Patenterteilung: **23.04.2014**
(21) Anmeldenummer: 08156639.0
(22) Anmeldetag: 08.11.2000
(51) Int. Cl.: C12N 15/11, C12N 15/12, C07K 7/08, C07K 14/435, C12N 15/62, C12N 15/63, C07K 16/40, C07K 1/14, C07K 1/04, A61K 38/10, A61K 31/7088, A61K 39/395, A61P 31/18

(54) **Humanes zirkulierendes Virus inhibierendes Peptid (VIRIP) und seine Verwendung**
Human circulating virus inhibitory peptide (VRIP) and its use
Peptide inhibiteur de virus humain (VIRIP) et son utilisation

(30) Priorität: 08.11.1999 DE 19953732; 16.05.2000 DE 10023665
(43) Veröffentlichungstag der Anmeldung: 20.08.2008
(62) Teilanmeldung aus: 00987221.9
(73) Patentinhaber: IPF Pharmaceuticals GmbH, 30625 Hannover (DE)
(72) Erfinder: Kirchhoff, Frank, 89081 Ulm (DE); Münch, Jan, 89231 Neu-Ulm (DE); Ständker, Ludger, 30625 Hannover (DE); Forssmann, Wolf-Georg, 79697 Wies-Wambach (DE); Adermann, Knut, 30177 Hannover (DE)
(74) Vertreter: von Kreisler Selting Werner

(56) Entgegenhaltungen:
- WO-A-84/02918
- WO-A-97/46100
- US-A- 5 420 110
- SHAPIRO LELAND ET AL: "Alpha-1-antitrypsin inhibits human immunodeficiency virus type 1." FASEB JOURNAL, Bd. 15, Nr. 1, Januar 2001 (2001-01), Seiten 115-122, XP002164955 ISSN: 0892-6638

### SEQUENCE LISTING

<110> IPF Pharmaceuticals GmbH
<120> Humanes zirkulierendes virus inhibierendes Peptid (VIRIP) und seine verwendung
<130> 080724ep
<160> 1
<170> PatentIn version 3.3
<210> 1
   <211> 20
   <212> PRT
   <213> Homo sapiens
<400> 1
   Leu Glu Ala Ile Pro Met Ser Ile Pro Pro Glu Val Lys Phe Asn Lys
   1               5                   10                  15
   Pro Phe Val Phe
               20

## Beschreibung

5 Gegenstand der vorliegenden Erfindung ist die Verwendung von Antikörpern gerichtet gegen VIRIP oder eines seiner Fragmente und/oder Derivate zu diagnostischen Zwecken, insbesondere bei Infektionen mit HIV-1.

WO 97/46100 A offenbart Antikörper gegen Alpha-1-Antitrypsin-Fragmente. WO 84/02918 A offenbart ein Polynucleotid, das für ein Peptid mit der Sequenz LEAIPMSIPPEVKFNKPFVF kodiert.

Die VIRIP konnte überraschenderweise mit Hilfe von chromatographischen Methoden und mit Hilfe eines biologischen Assays aus humanem Hämofiltrat isoliert werden. Die biochemische Charakterisierung des offenbarten Peptids erfolgte durch Massenspektrometrie und vollständigen Sequenzierung aller Aminosäuren.

Das Peptid besitzt die folgende Aminosäuresequenz:

Z₁ - LEAIPMSIPPEVKFNKPFVF - Z₂, worin Z₁, Z₂ unabhängig voneinander eine Anzahl von 0 bis zu 10 Aminosäureresten, und falls Z, oder Z₂=0 Aminosäurereste bedeuten, dann Z₁=H und/oder Z₂=COOH repräsentieren.

Die Molekularmasse des offenbarten Peptids VIRIP beträgt 2303 Da, wenn Z₁ und Z₂ keine Aminosäurereste bedeuten.

Als Derivate des VIRIP sind insbesondere amidierte, acetylierte, sulfatierte, Polyethylenglycol (PEG) modifizierte, phosphorylierte und/oder glykosylierte Derivate, sowie die durch multiple Synthese darstellbaren Peptide, welche die biologische Aktivität von VIRIP besitzen, zu nennen.

Das offenbarte Peptid umfasst ein 20 Aminosäuren umfassendes Fragment des bekannten humanen Proteins Alpha-1-Antitrypsin (Accession No. P01009), welches in seiner prozessierten Form aus 394 Aminosäuren besteht. Die Funktion des Alpha-1-Antitrypsin wird vornehmlich als Hemmstoff für die Enzyme Elastase sowie Thrombin und Plasmin beschrieben. Die offenbarte Peptidsequenz des VIRIP beginnt vorzugsweise hinter der Aminosäure 352 des Alpha-1-Antitrypsin und umfasst somit die Aminosäuren 353 bis 372 des Alpha-1-Antitrypsin.

Überraschenderweise bewirkt das offenbarte Peptid eine Unterdrückung der HIV-1-Infektion und/oder -Replikation von bzw. in humanen Blutzellen.

Das offenbarte Peptid ist durch ein Reinigungsverfahren ausgehend von humanem Hämofiltrat erhältlich. Dieses Hämofiltrat fällt bei der Ultrafiltration des Blutes von Nierenkranken in großen Mengen an.

Gegenstand der Erfindung sind Antikörper, die gegen die offenbarten Peptide gerichtet sind.

Das offenbarte Peptid kann auch mit einem Adapterprotein gekoppelt sein, das die Aufnahme in virusinfizierbare Zellen gewährleistet.

Offenbart werden auch Verfahren zur Behandlung von Patienten, die VIRIP benötigen durch Gabe therapeutischer Mengen der erfindungsgemäßen Polypeptide sowie Verfahren zur Behandlung von Patienten, die eine Inhibition des VRIP benötigen durch Gabe therapeutischer Mengen eines Antagonisten/Inhibitors.

Alternativ kann die therapeutische Wirkung des offenbarten Polypeptides durch Gabe von Polynukleotiden kodierend für VIRIP und anschließende Expression in vivo beim Patienten erreicht werden.

Das humane Hämofiltrat wird gegebenenfalls mit Wasser verdünnt und angesäuert. Der pH-Wert beträgt vorzugsweise 1,5 bis 3,5, insbesondere 2,5 bis 3,0. Danach wird das Hämofiltrat über einen Kationenaustauscher geleitet, beispielsweise einem mit Sulfonsäuregruppen modifizierenden Trägermaterial (Fraktogel SP - 650 (M), Merck, Darmstadt). Die an den Kationenaustauscher gebundenen Peptide werden mit einer relativ hoch konzentrierten Salzlösung eluiert. Die Ionenstärke der Elutionslösung entspricht dabei ungefähr einer 0,5 bis 1 molaren Ammoniumacetatlösung.

Das aufgefangene Eluat wird einer weiteren Kationenaustauscher-Chromatographie unterzogen. Diese Chromatographie ist vorzugsweise eine Stufenelution mit Puffern von ansteigenden pH-Werten.

Die das offenbarte Peptid enthaltenen Fraktionen werden mittels präparativer Umkehrphasen-Chromatographie und nachfolgender semipräparativer Umkehrphasen-Chromatographie beispielsweise an mit C18 modifizierten Trägermaterialien weitergereinigt. Der Aufreinigungsgrad wird vorzugsweise mittels analytischer Umkehrphasen-Chromatographie beispielsweise an mit C18 modifizierten Trägermaterialien überprüft.

Die durch die chromatographische Reinigung erhaltene Substanz wurde der Strukturaufklärung zugeführt. Die Bestimmung der Molekülmassen des gereinigten Peptids erfolgte mittels eines Elektrospray Massenspektrometers (ESI-MS). Die Sequenzanalyse des nativen Peptids erfolgte über einen Edman-Abbau mit einem ABI 473 A Sequenzer. Die offenbarte Peptidsequenz wurde chemisch synthetisiert und die Struktur des synthetisch hergestellten Peptids wurde ebenfalls aufgeklärt. Dieses synthetisch hergestellte VIRIP bewirkt ebenfalls eine dosis-abhängige Unterdrückung der HIV-1-Infektion und/oder -Replikation von bzw. in humanen Blutzellen.

Das offenbarte Peptid sowie seine cDNA, sein Gen und Analoga, Fragmente und Derivate zu dem Peptid, der cDNA und dem Gen sowie Antikörper, welche die Wirkung des VIRIP aufheben, können als Arzneimittel Verwendung finden. Seine biologische Aktivität entspricht der virus-inhibierender Substanzen. Dabei kann vermutet werden, dass VIRIP aufgrund seiner kurzen, hydrophoben Sequenz in die Blutzellen aufgenommen wird und dort als Hemmstoff von Virus-Enzymen oder als Hemmstoff von Enzymen der Blutzellen wirkt oder dass VIRIP an Rezeptoren bindet, welche für den Eintritt von Viren von Bedeutung sind. Somit verhindert VIRIP die Infektion von Zellen mit dem Virus.

Das erfindungsgemäße Diagnostikmittel enthält poly- oder monoklonale Antikörper gegen das erfindungsgemäße Peptid gegebenenfalls in fluoreszenz-oder radioaktiv-markierter Form, um in einem an sich bekannten ELISA oder RIA eingesetzt zu werden. Die erfindungsgemäß verwendbaren Antikörper gegen das Peptid können durch Ummunisierung von Säugetieren erhalten werden.

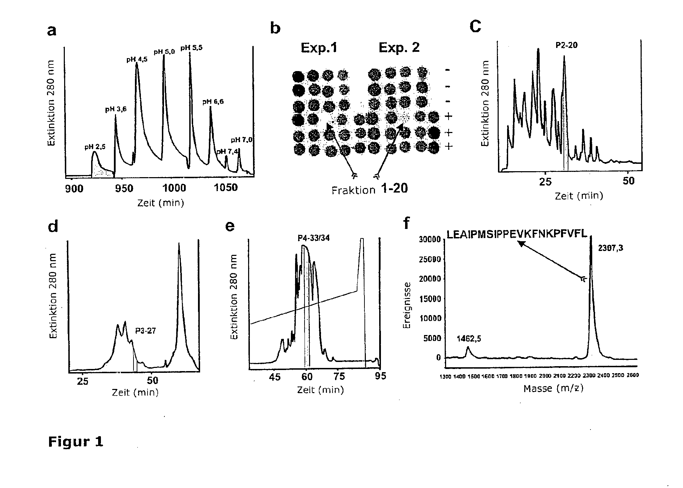

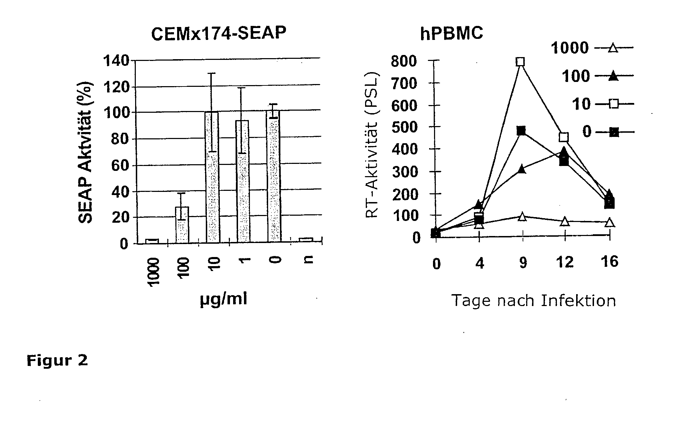

Figur 1: Aufreinigung von VIRIP. Die Aufreinigungsschritte sind im Text beschrieben. (b) Inhibition der Replikation von HIV-1 NL43 in humanen Blutlymphozten durch die Fraktion 1-20. Die Zellen wurden 2 Tage mit PHA stimuliert, mit Virusstocks die 10 ng p24 enthalten infiziert und die Reverse Transkriptase-Aktivität im Kulturüberstand 9 Tage nach Infektion bestimmt. Dargestellt ist das Ergebnis der Infektionsexperimente. Figur 2: Einfluss von synthethischem VIRIP auf die Infektion von CEMx174-SEAP Indikatorzellen (links) und die Replikation in humanen PBMCs (rechts). Die Zellen wurden in Gegenwart der aufgeführten Mengen an VIRIP infiziert und kultiviert. Zur Bestimmung des viralen Eintritts wurde die Aktivität der sekretierten Alkalinen Phosphatase in Kulturüberstand der CEMx174-SEAP Zellen drei Tage nach der Infektion bestimmt.

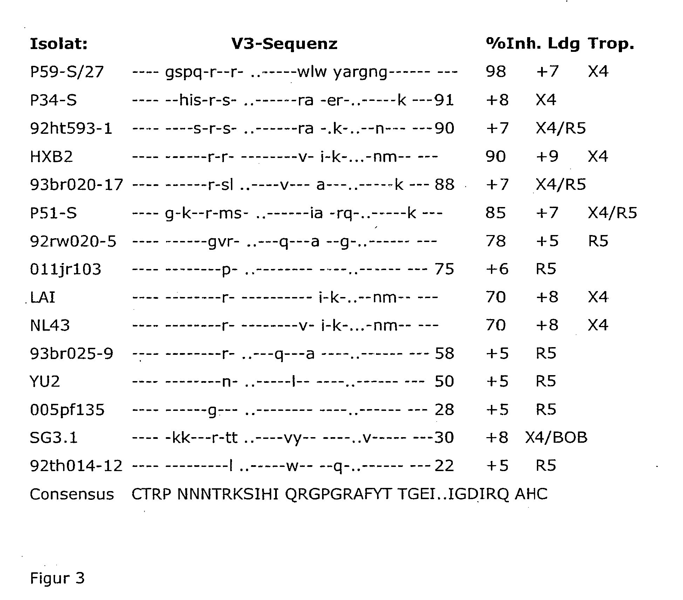

Figur 3: Die V3-Schleife des X4-tropen NL43 Klons wurde gegen die entsprechenden Sequenzen der dargestellten HIV-1 Isolate ausgetauscht. Die rekombinanten Viren unterscheiden sich somit ausschließlich in der V3-Schleife. Im rechten Panel ist der beobachtete inhibitorische Effekt (%), die Gesamtladung der V3-Region und der Korezeptortropismus dargestellt.

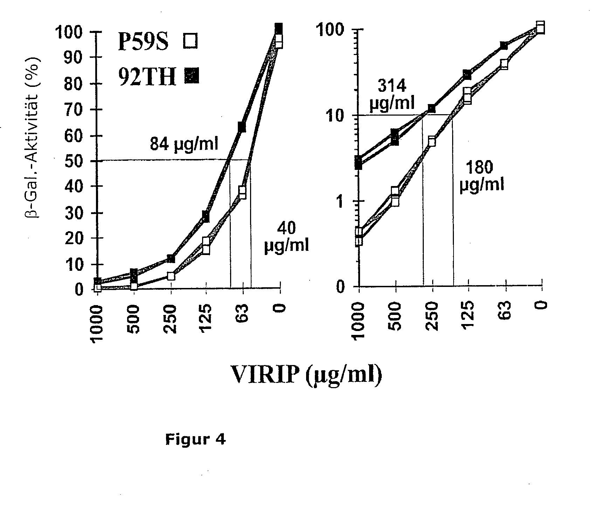

Figur 4: Dosis-abhängige Hemmung der X4-tropen P59S und der R5-topen 92TH HIV-1 NL4-3 Rekombinanten durch VIRIP. P4R5 Indikatorzellen wurden in Gegenwart der dargestellten Konzentrationen an VIRIP infiziert und die β-Galaktosidase-Aktivität im Zellextrakt 2 Tage nach Infektion gemessen.

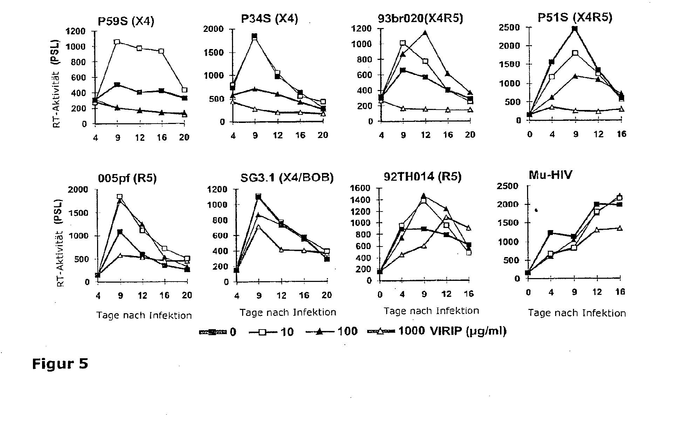

Figur 5: VIRIP hemmt die Vermehrung verschiedener HIV-Isolate mit unterschiedlichem Zell- und Korezeptor-Tropismus in humanen PBMC. Vorstimulierte PBMC wurden in Gegenwart der dargestellten VIRIP Konzentrationen mit den diversen HIV-1 Isolaten (10 ng p27 antigen) oder einem chimären Onko-Lentivirus (Mu-HIV). Die Virusvermehrung wurde durch die Messung der Reverse Transkriptase-Aktivität im Zellkulturüberstand bestimmt.

Die Erfindung wird anhand der folgenden nicht erfindungsgemäßen Beispiele näher beschrieben.

### Beispiel 1

### Isolierung des antiviral wirksamen VIRIP

### 1. Schritt: Hämofiltrat-Batch-Extraktion

800-1000 L Hämofiltrat werden mit HCl auf einen pH -Wert von 2.7 eingestellt und mit Wasser auf eine Leitfähigkeit von 5.5 mS/cm verdünnt und mit einer Flussrate von 3 L/min auf einen starken Kationenaustauscher aufgetragen.

### Chromatographiebedingungen:

| | |
|---|---|
| Säule: | Vantage VA 250 (Amicon, Witten) |
| Säulenmaterial: | Fractogel TSK SP 650 (M), 25 cm x 20 cm |
| Fluss: | 3 L/min |
| Detektion: | 280 nm, pH, Leitfähigkeit |
| Puffer A: | Hämofiltrat pH 2.7, Leitfähigkeit 5.5 mS/cm |
| Puffer B: | 0.5 M Ammoniumacetat |
| Anlage: | Autopilot Chromatographiesystem, (PerSeptive Bio systems, Wiesbaden) |

Nach Auftrag der insgesamt 1.000 L Flüssigkeit über Nacht wird mit mehreren Säulenvolumina 5 mM HCl gespült. Die Elution der gebundenen Peptide erfolgt als Batch-Elution mit 0.5 M Ammoniumacetat. Hierbei wird eine komplette Elution der Peptide über steigenden pH-Wert (6.8 - 7.2) und steigende Leitfähigkeit (56 mS/cm) in etwa 5 L Eluat erreicht.

### 2. Schritt: Erste präparative Auftrennung (Charge 01/1998)

Die Ammoniumacetat-Eluate der Batch-Extraktion werden in einer Menge von 10.000 L Hämofiltrat-Peptid vereinigt. Nach pH-Einstellung auf 2.7 wird das Peptidextrakt unter Zumischung von VE-Wasser mit einer Leitfähigkeit von 5.5 mS/cm auf den präparativen Kationenaustauscher aufgetragen.

### Chromarographiebedingungen:

| | |
|---|---|
| Säule: | Vantage 250 VA |
| Säulenmaterial: | Fractogel TSK SP 650 (M), 25 cm x 20 cm |
| Fluss: | bis zu 3 L/min während des Auftrages 0.5 bis 1 L während der Elution |
| Detektion: | 280 nm, pH, Leitfähigkeit |
| Probe: | Hämofiltrat pH 2.7, Leitfähigkeit 5.5 mS/cm |
| Anlage: | Autopilot Chromatographiesystem, (PerSeptive Bio systems, Wiesbaden) |

Nach Auftrag des Rohextraktes über 240 min wird die Säule mit 0.01 M HCl gespült, bis die Leitfähigkeit unter 1 mS/cm ist. Die Elution erfolgt dabei in mehreren Stufen mit den im folgenden angegebenen Puffern

| Puffer | pH-Wert | Puffersubstanzen | Leitfähigkeit (mS/cm) |
|---|---|---|---|
| Waschpuffer: | 2.0 | 0.01 M HCl | 1 |
| Elutionspuffer 1: | 3.6 | 0.1 M Zitronensäure-1-hydrat | 2.9 |
| Elutionspuffer 2: | 4.5 | 0.1 M Essigsäure + 0.1 M Natriumacetat | 4.0 |
| Elutionspuffer 3: | 5.0 | 0.1 M Äpfelsäure | 6.2 |
| Elutionspuffer 4: | 5.6 | 0.1 M Bernsteinsäure | 6.1 |
| Elutionspuffer 5: | 6.6 | 0.1 M NaH₂PO₄ | 4.9 |
| Elutionspuffer 6: | 7.4 | 0.1 M NaH₂PO₄ | 6.7 |
| Elutionspuffer 7: | 9.0 | 0.1 M Ammoniumcarbonat | 6.7 |

Die Eluate 1-7 werden als pH-Pool I-VII bezeichnet (s. Fig. 1a). Sie werden separat gesammelt und abschließend mit VE-Wasser gespült. Die Elution erfolgt bis zum Erreichen einer neuen Basislinie, wobei für die einzelnen pH-Pools I bis VII Elutionsvolumina von 10 bis 25 L erreicht werden.

### 3. Schritt: Zweite präparative Auftrennung:

Die einzelnen pH-Pools werden zur Fraktionierung und gleichzeitigen Entsalzung über eine Reversed Phase Chromatographie getrennt

### Chromatographiebedingungen:

| | |
|---|---|
| Säule: | FineLine 100 (Pharmacia, Freiburg) |
| Säulenmaterial: | Source RPC, 15 µm |
| | 10 x 12.5 cm (FineLine 100) |
| Fluss: | 150 mL/min (FineLine 100) |
| Detektion: | 280 nm, Leitfähigkeit, pH |
| Puffer A: | 10 mM HCl |
| Puffer B: | 80% Acetonitril in 10 mM HCl |
| Gradient: | 0-60% Puffer B in 5 Säulenvolumen |

Nach Auftrag der einzelnen pH-Pools wird die Säule mit Puffer A gespült. Während der Elution werden Fraktionen zu 200 ml gesammelt. Die Fraktionen werden gefriergetrocknet und bei -20°C gelagert. Aliquots der entstandenen Fraktionen werden im Bioassay getestet. Die Fraktion 20 aus pH-Pool I enthielt das erfindungsgemäße Peptid (s. Fig. 1b, c).

### 4. Schritt: Semipräparative Reverse-Phase C18-Chromatographie:

Insgesamt 500 mg der im Assay bioaktiven Fraktion 20 aus pH-Pool I wurden über eine semipräparative Reverse-Phase Säule aufgetrennt. Die Fraktion 27 enthielt die erfindungsgemäße Substanz (s. Fig. 1d).

### Chromatographiebedingungen:

| | |
|---|---|
| Säule: | 4,7 cm x 30 cm Stahlsäule |
| Füllmaterial: | Vydac RP-C18 15-20 µm, 300 Ä |
| Puffer A: | 30% Methanol, 70% Wasser, 0.1% TFA |
| Puffer B: | 100% Methanol, 0.1% TFA |
| Gradient: | 0 - 60% B in 2100 ml |
| Fluss: | 40 ml/min |
| Detektion: | 214 nm und 280 nm |
| Chromatographieanlage: | BioCad 250, Perseptive Biosystems |
| Fraktionen: | à 50 ml ab Start des Gradienten |

### 5. Schritt: Analytische Reverse-Phase C4-Chromatographie:

Die bioaktive Fraktion 27 aus der vorhergehenden Chromatographie wurde über eine analytische Reverse-Phase Säule aufgetrennt. Aliquots wurden im Bioassay getestet. Die Fraktionen 33+34 enthielten die erfindungsgemäße Substanz in aufgereinigter Form.

### Chromatographiebedingungen: 5 µm, 100 Å, 20 x 250 mm;

| | |
|---|---|
| Säule: | 2 cm x 25 cm Stahlsäule |
| Füllmaterial: | RP-C4, 5 µm, 100 Ä, Biotek Silica, Östringen, Germany |
| Puffer A: | Wasser, 0.1 % TFA |
| Puffer B: | 80 % Acetonitril, 20% Wasser, 0.1 % TFA, |
| Gradient: | 20 - 60% B in 80 min, 60 - 100% B in 2 min |
| Fluss: | 8 ml/min |
| Detektion: | 214 nm und 280 nm |
| Chromatographieanlage: | Kontron |
| Fraktionen: | à 1.5 min ab Start des Gradienten |

Die erfindungsgemäße Reinsubstanz wurde daraufhin in dosisabhängiger Weise im Bioassay untersucht und peptidchemisch charakterisiert.

### Beispiel 2

### Massenbestimmungen

Die Massenbestimmungen des aus Hämofiltrat isolierten Peptids (aus den Fraktionen 33+34 des 5. Schritts in Beispiel 1) und des chemisch synthetisierten Peptids (Beispiel 3) wurden auf einem ESI Massenspektrometer durchgeführt (s. Fig. 1f). Die Molekülmasse der Peptide wurden entsprechend der nachfolgend gezeigten Massenzahlen (MW) bestimmt:

| | |
|---|---|
| VIRIP, isioliert aus humanem Hämofiltrat: | 2303 Da |
| VIRIP, chemisch synthetisiertes Peptid: | 2303 Da |

### Sequenzbestimmung

Die aufgereinigten nativen und chemisch synthetisierten Peptide werden mittels Edman-Abbau auf einem ABI 473 A Sequenzer unter Verwendung des Standard-Programms analysiert. Die Proben werden auf eine Polybrene-Membran in Mengen zwischen 100 und 400 pmol aufgetragen. Es ergaben sich sowohl für das aus Hämofiltrat isolierte Peptid (aus den Fraktionen 33+34 des 5. Schritts in Beispiel 1) und das chemisch synthetisierte Peptid (Beispiel 3) folgende identische vollständige Aminosäuresequenz:

### LEAIPMSIPPEVKFNKPFVF

### Datenbankvergleich

Ein Datenbankvergleich wurde mit Hilfe des HUSAR-Programmpakets an den SwissProt und EMBL-Nukleinsäure Datenbanken durchgeführt. Die Peptidsequenz besitzt eine hundertprozentige Identität zu dem aus der cDNA abgeleiteten Aminosäuren 353-372 des humanen Proteins Alpha-1-Antitrypsin (Accession No. P01009).

### Beispiel 3

### Chemische Synthese von VIRIP

Die chemische Synthese von VIRIP wurde mittels konventioneller Festphasensynthese auf einem Peptid-Synthesizer 9050 unter Verwendung der bekannten Fmoc-Chemie durchgeführt. Das erhaltene Peptid wurde über Reverse-Phase Chromatographie aufgereinigt, seine Identität und Reinheit wurde mittels analytischer RP-HPLC sowie der unter Beispiel 2 beschriebenen Massen-und Sequenzbestimmung festgestellt.

### Beispiel 4

### Bestimmung der antiviralen Aktivität von VIRIP

Die Isolierung der VIRIP erfolgte aufgrund ihrer inhibitorischen Wirkung auf die Vermehrung von HIV-1 in einem Assay, der die Replikation von HIV-1 in peripheren humanen Blutlymphozyten (PBMCs) misst. Dazu wurden jeweils Aliquots der unter Beispiel 1 beschriebenen einzelnen Chromatographiestufen gefriergetrocknet und anschließend dem biologischen Assay in Mengen von 10 ml bis zu 1 L Hämofiltrat-Äquivalent zugeführt. Die Fraktionen, die jeweils ein positives Signal ergaben, wurden der weiteren Aufreinigung unterzogen.

Humane periphere Blutlymphozyten (PBMCs) wurden aus Vollblut mittels Dichtegradienten-Zentrifugation in Ficoll gewonnen. Die Zellen wurden für zwei Tage vorstimuliert (RPMI-Medium, 20% FKS, 100 U/ml IL-2, 5 µg/ml Phytohämagglutinin [PHA]). Anschließend wurden die PBMCs durch Zentrifugation für 5 min bei 1200 rpm sedimentiert, in PHA-freiem RPMI-Medium aufgenommen (20% FKS, 100 U/ml IL-2) und in einer Konzentration von etwa 150.000 Zellen pro Loch in 96-Well-Flachboden-Platten in einem Volumen von 80 µl ausgesät.

Am folgenden Tag wurden Aliquots der unter Beispiel 1 beschriebenen einzelnen Chromatographiestufen in Mengen von 10 ml bis zu 1 L Hämofiltrat-Äquivalent zugeführt. Dazu wurden die gefriergetrockneten Chromatographiestufen in Wasser aufgenommen und in verschiedenen Konzentrationen in einem Gesamtvolumen von 10 µl zu den PBMCs pipettiert. Nach 2stündiger Inkubation bei 37°C wurden die Zellen durch die Zugabe von 10 µl HIV-1 Virusstock, der 0,1 bis 10 ng p24 Antigen enthält, infiziert. Im folgenden wurden 50 µl des Kulturmediums alle 2 Tage durch frisches Medium ersetzt, welches zusätzlich die korrespondierenden Peptid-Fraktionen enthält. Die Produktion an HIV-Partikeln zu verschiedenen Zeitpunkten (9, 12 und 18 Tage) nach der Infektion der Zellen wurde im Reverse-Transkriptase(RT)-Test quantifiziert. Der RT-Test misst die Aktivität der Reversen-Transkriptase im zellfreien Kulturüberstand und ist somit ein Maß für die Produktion an Viruspartikeln und die virale Vermehrung in den PBMC-Kulturen. Alternativ wurde die Virusproduktion im p24 Antigen-ELISA bestimmt. Fraktionen, die im Vergleich zu Ansätzen ohne potentiell stimulierende oder inhibitorische Peptide zu einer mindestens 90%igen Reduktion der RT-Produktion führten (s. z.B. Fig. 1b) und somit die Vermehrung von HIV-1 in humanen PBMC effizient hemmten, wurden weiter aufgereinigt.

Das aus Hämofiltrat aufgereinigte VIRIP (Beispiel 1) als auch das chemisch synthetisierte VIRIP (Beispiel 3) zeigten eine dosisabhängige Inhibition der HIV-1 Infektion von CEMx174-SEAP Indikatorzellen und der Replikation in PBMCs (Fig. 2). Das erfindungsgemäße VIRIP besitzt dagegen keine zytotoxische Wirkung auf die Blutzellen.

### Beispiel 5. Die Effizienz der VIRIP-spezifischen Inhibition ist abhängig von der Sequenz der V3-Schleife im HIV-1 Hüllprotein.

Die Ergebnisse belegten, dass sowohl VIRIP aus Hämofiltrat, als auch das synthetisch hergestellte Peptid die Replikation von HIV-1 NL43 wirksam blockieren. Um herauszufinden, ob VIRIP spezifisch für X4-trope Varianten ist oder auch andere Formen inhibiert, welche den Korezeptor CCR5 benutzen oder dual-trop (X4/R5) sind, wurde eine Reihe von V3-Varianten des molekularen NL4-3 Klons untersucht (Fig. 3). Der V3-Loop des X4-tropen NL4-3 Klons wurde durch die entsprechende Region einer Reihe von primären HIV-1 Isolaten ersetzt. Funktionelle Tests mit verschiedenen Indikatorzellinien zeigten, dass diese V3-Rekombinanten einen unterschiedlichen Zell- und Korezeptor-Tropismus aufweisen. Um den inhibitorischen Einfluss von VIRIP auf diese Varianten zu untersuchen wurden P4R5 Indikatorzellen verwendet. P4R5-Zellen exprimieren sowohl CCR5 als auch CXCR4 und enthalten das Luziferasegen unter der Kontrolle der HIV-1 LTR. Diese Zellinie wurde in Gegenwart und Abwesenheit von VIRIP mit den diversen rekombinanten Viren infiziert (50 ng p24) und die Infektion mit Hilfe des Luziferasetest quantifiziert. Wie in der Figur 3 dargestellt inhibierte VIRIP sowohl X4-, als auch R5- und X4/R5-trope Varianten. Insgesamt wurden jedoch X4-trope Varianten mit stark positiv geladenem V3-Loop effizienter gehemmt als R5- oder dual-trope Isolate.

Um den inhibitorischen Effekt der erfindungsgemäßen Substanz genauer zu quantifizieren, wurden P4R5 Indikator Zellen in Gegenwart verschiedener Dosen von VIRIP mit dem X4-tropen HIV-1 P59S und dem R5-tropen 92TH Isolat infiziert. Wir in der Fig. 4 dargestellt, inhibierte VIRIP das HIV-1 P59S Isolat bei einer Konzentration von 40 µg/ml um 50%, und bei einer Konzentration von 180 µg/ml um 90%. Zur Hemmung des X-tropen 92TH Isolates waren etwa 2-fach höhere Konzentrationen an VIRIP erforderlich (Fig. 4).

In weiteren Experimenten wurde gezeigt, dass VIRIP die Replikation von X4 - tropen und dual-tropen HIV-Varianten in humanen PBMC bei Konzentrationen von 1000 µg/ml komplett und bei Konzentrationen von 100 µg/ml zumindest partiell unterdrückt (Fig. 5, oben). Die inhibitorischen Effekte auf R5-trope Formen waren geringer. Ein chimäres Onko-Lentivirus (Mu-HIV), welches das MLV-Hüllprotein trägt, wurde nicht inhibiert (Fig. 5, unten).

## Patentansprüche

1. Verwendung von Antikörpern gerichtet gegen ein Peptid mit nachfolgender Aminosäuresequenz:
LEAIPMSIPPEVKFNKPFVF (VIRIP)
sowie Fragmente und/oder amidierte, acetylierte, sulfatierte, Polyethylenglycol (PEG) modifizierte, phosphorylierte und/oder glykosylierte Derivate, welche die Infektion und/oder Replikation von bzw. in humanen Blutzellen durch HIV-1 unterdrücken, für Testsysteme zur Kontrolle von Gewebe-, Plasma-, Urin- und Liquor cerebrospinalis-Spiegeln von VIRIP.

2. Verwendung gemäß Anspruch 1 als Marker für virale Erkrankungen, bakterielle und Pilz-Infektionen, inflammatorische und neoplastische Prozesse sowie als Marker bei entzündlichen Prozessen, gestörten Entzündungsreaktionen, Tumorerkrankungen, Wachstumsstörungen, Erkrankungen des Immunsystems sowie als Marker bei Knochenerkrankungen.

## Claims

1. Use of antibodies directed against a peptide having the following amino acid sequence:
**LEAIPMSIPPEVKFNKPFVF (VIRIP)**
as well as fragments and/or amidated, acetylated, sulfated, polyethylene glycol (PEG) modified, phosphorylated and/or glycosylated derivatives thereof that suppress the infection of human blood cells by HIV-1 and/or the replication of HIV-1 in human blood cells, for test systems for monitoring levels of VIRIP in tissue, plasma, urine and cerebrospinal fluid.

2. The use according to claim 1 as a marker for viral diseases, bacterial and fungal infections, inflammatory and neoplastic processes, and as a marker in inflammatory processes, disturbed inflammatory responses, tumor diseases, growth disorders, diseases of the immune system, and as a marker in bone diseases.

## Revendications

1. Utilisation d'anticorps dirigés contre un peptide avec la séquence d'acides aminés suivante :
LEAIPMSIPPEVKFNKPFVF (VIRIP),
ainsi que des fragments et/ou des dérivés amidifiés, acétylés, sulfatés, modifiés par du poly(éthylène glycol) (PEG), phosphorylés et/ou glycosylés, qui suppriment l'infection et/ou la réplication par le HIV-1, respectivement, de cellules sanguines humaines ou dans des cellules sanguines humaines, pour des systèmes de test destinés à contrôler les taux de VIRIP dans les tissus, le plasma, l'urine et le liquide céphalorachidien.

2. Application selon la revendication 1, comme marqueur pour les maladies virales, les infections mycosiques ou bactériennes, les processus inflammatoires et néoplasiques, ainsi que comme marqueur pour les processus d'inflammation, les réponses inflammatoires perturbées, les affections tumorales, les troubles de la croissance, les maladies du système immunitaire, ainsi que comme marqueur pour les maladies osseuses.
